# EUROPEAN PATENT SPECIFICATION

(11) **EP 4 347 110 B1**
(45) Date of publication and mention of the grant of the patent: **08.07.2026**
(21) Application number: 22816709.4
(22) Date of filing: 31.05.2022
(51) Int. Cl.: B01J 19/00, B82Y 30/00, C25B 11/02, C25B 3/00

(54) **ELECTROGRAFTED FILMS FOR DNA SYNTHESIS**
ELEKTROGEPFROPFTE FILME ZUR DNA-SYNTHESE
FILMS ÉLECTROGREFFÉS POUR LA SYNTHÈSE D'ADN

(30) Priority: 31.05.2021 WO PCT/CN2021/097127
(43) Date of publication of application: 10.04.2024
(73) Proprietor: Genscript USA Inc., Redmond, WA 98052 (US); CustomArray, Inc., Redmond, Washington 98052 (US); Nanjing GenScript Biotech Co., Ltd., Nanjing, Jiangsu 211100 (CN)
(72) Inventor: HU, Feichi, Nanjing, Jiangsu 211100 (CN); REED, Michael W., Redmond, Washington 98052 (US); COOPER, John, Redmond, Washington 98052 (US); KELLOCK, Jackson, Redmond, Washington 98052 (US); WU, Cheng-Hsien, Redmond, Washington 98052 (US)
(74) Representative: Michalski Hüttermann & Partner mbB
(86) International application number: PCT/US2022/031555
(87) International publication number: WO 2022/256303

(56) References cited:
- US-A1- 2006 141 156
- US-A1- 2009 093 381
- US-A1- 2013 203 631
- US-A1- 2021 106 967
- US-B1- 6 251 595
- US-B1- 6 262 040
- US-B2- 10 525 436
- DOYLE ET AL.: "Alkyl Nitrite-Metal Halide Deamination Reactions. 6. Direct Synthesis of Arenediazonium Tetrafluoroborate Salts from Aromatic Amines, tert-Butyl Nitrite, and Boron Trifluoride Etherate in Anhydrous Media", J. ORG. CHEM., vol. 44, no. 9, 1979, pages 1572 - 1574, XP002335096, DOI: 10.1021/jo01323a048
- JIANG CHENG, ALAM MUHAMMAD TANZIRUL, PARKER STEPHEN G., DARWISH NADIM, GOODING J. JUSTIN: "Strategies To Achieve Control over the Surface Ratio of Two Different Components on Modified Electrodes Using Aryldiazonium Salts", LANGMUIR, AMERICAN CHEMICAL SOCIETY, US, vol. 32, no. 10, 15 March 2016 (2016-03-15), US , pages 2509 - 2517, XP093014558, ISSN: 0743-7463, DOI: 10.1021/acs.langmuir.5b04550

## Description

### TECHNICAL FIELD OF THE INVENTION

The present disclosure provides films formed by electrografting of functionalized aryldiazonium salts. Methods for oligonucleotide synthesis utilizing electrodes coated with thin films formed by electrografting of functionalized aryldiazonium salts are also provided.

### BACKGROUND OF THE INVENTION

Rapid developments in the field of DNA microarrays have led to a number of methods for synthetic preparation of DNA. Such methods include spotting pre-synthesized oligonucleotides, photolithography using mask or maskless techniques, in *situ* synthesis by printing reagents, and *in situ* parallel synthesis on a microarray of electrodes using electrochemical deblocking of protective groups. A review of oligonucleotide microarray synthesis is provided by, for example, Gao et al., Biopolymers 2004, 73:579. The synthetic preparation of a peptide array was reported in 1991 using photo-masking techniques. This method was extended in 2000 to include an addressable masking technique using photogenerated acids and/or in combination with photosensitizers for deblocking. Reviews of peptide microarray synthesis using photolabile deblocking are provided by: Pellois et al., J. Comb. Chem. 2000, 2:355 and Fodor et al., Science, 1991, 251:767. Spotting pre-synthesized peptides or isolated proteins has been used to create peptide arrays. A review of protein or peptide arrays is provided by: Cahill and Nordhoff, Adv. Biochem. Engin/Biotechnol. 2003, 83:177.

Electrochemical parallel DNA synthesis on CMOS (complementary metal oxide semiconductor) type electrode arrays has been described (Maurer et. al, 2006, PLoS One. 2006 Dec 20; 1(1):e34; U.S. Patent No. 10,525,436). In this application, the CMOS chip surface is coated before DNA synthesis with an absorbed porous reaction layer over each platinum electrode. DNA synthesis starts from hydroxyl groups on a porous layer. After DNA synthesis, the oligo is either left on the chip or the oligo is cleaved from the chip surface. One problem with the absorbed porous coating is the long cleavage time for release of the oligo. The long cleavage time slows DNA synthesis production and may affect DNA quality. The released oligos may still have an absorbed coated molecule linked to the 3'-terminus. 3'-modification of the DNA strand is a problem for some applications (such as PCR primers) since 3'-modification blocks polymerase extension. Another problem with the absorbed coating on electrodes is degradation during DNA synthesis or when used for multiple hybridization assays.

Chemistry has been described for coating gold electrodes with phenylethanol groups for DNA synthesis (Levrie, 2018, Jpn. J. Appl. Physics, 04FM01) in which the diazonium salt was prepared from 3 mM 4-aminophenyl alcohol (APE-OH) "in situ" using excess sodium nitrite in acid.

Similarly, APE-OH diazonium (Dz) salts have been deposited on gold surfaces and further functionalized to make polymer grafts for molecular imprinting (Gam-Derouich, 2010, Surf. Int. Anal. 1050).

Although aryldiazonium electrografting onto platinum electrode surfaces has been reported (Bernard, et. al, 2003, Chem. Mater. 3450-3462), the surface coatings did not have suitable functional linker groups for DNA synthesis or attachment of biomolecules using standard chemical coupling methods.

The present invention addresses these challenges by providing methods for synthesis of oligonucleotides utilizing functionalized aryldiazonium salts to coat microelectrodes in CMOS chips.

### BRIEF SUMMARY OF THE INVENTION

Provided herein are methods and compositions for oligonucleotide synthesis utilizing electrodes coated with thin films formed by electrografting of functionalized aryldiazonium salts.

The invention is defined by the features of the appended independent claims. Preferred embodiments are defined by the features of the dependent claims.

In particular, the present invention relates to
A solid support system for synthesis of oligonucleotides, wherein the support is an electrode coated with a thin film comprising polyalcohol, wherein the electrode is prepared by a method comprising
(a) cleaning the electrode surface,
(b) introducing a diazonium salt to the cleaned electrode surface,
(c) electrografting the diazonium salt so as to form a multi-layer film of polyalcohol onto the cleaned electrode surface,
   wherein the multi-layer film comprises a loose outer layer of the polyalcohol and an inner stable layer of the polyalcohol,
(d) stripping the loose outer layer of the polyalcohol, and
(e) obtaining the inner stable layer of the polyalcohol for the synthesis of oligonucleotides.

The present invention further relates to
A method of preparing an electrode surface for oligonucleotide synthesis, comprising
(a) cleaning the electrode surface,
(b) introducing a diazonium salt to the cleaned electrode surface,
(c) electrografting the diazonium salt so as to form a multi-layer film of polyalcohol onto the cleaned electrode surface,
   wherein the multi-layer film comprises a loose outer layer of polyalcohol and an inner stable layer of polyalcohol,
(d) stripping the loose outer layer of polyalcohol, and
(e) performing the oligonucleotide synthesis on the inner stable layer of polyalcohol.

In one embodiment, functionalized aryldiazonium salts are used to introduce a thin film comprising functional groups (such as a polyalcohol film) directly onto the surface of the electrode for DNA synthesis to initiate. Functionalized aryldiazonium salts according to this embodiment have the formula:

Y-R-Ar-N₂⁺X⁻,

wherein:
- Y: is a functional group;
- R: is any organic group or is absent; and
- X: is an inorganic or organic anion.

In some embodiments, Ar is selected from phenyl, xylyl, naphthyl, tolyl, and indolyl. In some embodiments, Ar is phenyl.

In some embodiments, Y is -OH, -COOH, or -NH₂. In some embodiments, Y is protected. For example, in some embodiments, Y is protected -OH, protected-COOH, or protected -NH₂.

In some embodiments, R is selected from an alkyl group, an alkenyl group, an aryl group, and a heteroaryl group. In some embodiments, R is selected from a linear or branched C₁-C₁₆ alkyl group, a linear or branched C₁-C₁₂ alkyl group, a linear or branched C₁-C₈ alkyl group. In some embodiments, R is selected from a methyl, ethyl, isopropyl, n-propyl, tert-butyl, isobutyl, n-butyl, n-pentyl, isoamyl and 1,1-dimethylpropyl group.

In some embodiments, X is a halogen, tetrafluoroborate, or tosylate.

In certain embodiments, the functionalized aryldiazonium salts are selected from the group consisting of: 4-(hydroxymethyl)benzenediazonium tetrafluoroborate salt (AB-OH diazonium salt), 4-(2-hydroxyethyl)benzenediazonium tetrafluoroborate salt (APE-OH diazonium salt), and 4-carboxybenzenediazonium tetrafluoroborate salt (AB-acid diazonium salt).

In some embodiments, the functionalized aryldiazonium salts are purified.

Exposed alcohol residues on the surface of the film react with an activated (dimethoxytrityl (DMT) protected) nucleoside phosphoramidite during the first DNA synthesis coupling cycle. Unreacted alcohol residues on the surface of the film are further capped during synthesis and the oligonucleotide strand is further assembled. The polyalcohol coated electrodes can be further functionalized with cleavable linkers, to allow release of DNA after synthesis.

In one aspect, the invention provides a solid support system for synthesis of oligonucleotides, wherein the support is an electrode coated with a thin film comprising functional groups. In some embodiments, the thin film comprises carboxylic acid and/or alcohol functional groups. In some embodiments, the thin film comprises polyalcohol, preferably polybenzyl alcohol. In some embodiments, the thin film is formed by electrografting of functionalized aryldiazonium salts having the formula:

Y-R-Ar-N₂⁺X⁻,

wherein:
- Y: is a functional group;
- R: is any organic group or is absent; and
- X: is an inorganic or organic anion.

In some embodiments, the thin film comprises at least two layers. In certain embodiments, the thin film comprises a loose outer layer of polyalcohol (e.g. polybenzyl alcohol) and a stable inner hydroxyl layer. In some embodiments, the loose outer layer of polyalcohol (e.g. polybenzyl alcohol) can be removed with concentrated ammonium hydroxide.

In further embodiments, the solid support is further modified with long spacer phosphoramidite molecules and/or cleavable linkers. In some embodiments, the spacer can be branching amines or polymeric amines, such as PEI or poly-L-lysine.

In some embodiments, the solid support is an electrode array device, optionally fabricated using standard CMOS technology. In certain embodiments, the electrode is a platinum, titanium or titanium nitride electrode.

Also disclosed herein is a method of coating a solid support system for synthesis of oligonucleotides with a thin film comprising a functional group (such as a carboxylic acid or alcohol functional group), the method comprising treating the solid support with a solution comprising an aryldiazonium salt and an electrolyte and optionally energizing the solid support, thereby producing a coated solid support. In some embodiments, the coated solid support is further reacted with additional reactive free radical of diazonium monomer groups to give a multi-layer film. In some embodiments, the multi-layer film comprises a loose outer layer of polyalcohol (e.g. polybenzyl alcohol) and a stable inner hydroxyl layer. In some embodiments, the method of coating a solid support system for synthesis of oligonucleotides further comprises washing the coated solid support with acetonitrile. In some embodiments, the method of coating a solid support system for synthesis of oligonucleotides further comprises treating the coated solid with concentrated ammonium hydroxide. In some embodiments, the support is an electrode, optionally a platinum, titanium or titanium nitride electrode. In certain embodiment, the support is a platinum electrode. In certain embodiment, the aryldiazonium salt is selected from the group consisting of: 4-(hydroxymethyl)benzenediazonium tetrafluoroborate salt (AB-OH diazonium salt), 4-(2-hydroxyethyl)benzenediazonium tetrafluoroborate salt (APE-OH diazonium salt), and 4-carboxybenzenediazonium tetrafluoroborate salt (AB-acid diazonium salt).

In further embodiments, the methods and compositions may comprise a solid support system for synthesis of oligonucleotides, wherein the support comprises electrodes coated with thin films containing functional groups. In some embodiments, the functional groups are carboxylic acid and/or alcohol groups. Carboxylic acid groups can be further reacted with aminoalcohol spacer molecules with the goal of extending the terminal alcohol away from the solid surface. Branching amines or polymeric amines such as PEI or poly-L-lysine can also be immobilized to the electrode surface using this method to vary the structure of the amine coat on the electrode surface. This allows surface density of amine groups to be further controlled. Amine coated solid supports are routinely used for automated DNA synthesis. Similar methods can be used to introduce cleavable linkers or spacer molecules to improve yields and quality of DNA synthesis on amine-coated electrodes.

In an embodiment, methods for synthesis of oligonucleotides may comprise using the compositions described herein. DNA synthesis applications require high yields of full-length product with length of 10-200 nt being valuable. In one application, pools of oligonucleotides are synthesized in microarray format with a cleavable linker at the 3'-terminus of the designed sequence. After synthesis, the DNA strand is subsequently cleaved from the chip and deprotected to give a "pool" of the oligonucleotides in solution. For pooled oligos, a less dense (loose) surface structure on the film provides more available hydroxyl groups to initiate DNA synthesis over each electrode (high yields after DNA synthesis). However, this "loose layer" of film is fragile and not suitable for DNA synthesis applications that require microarrays of probes to remain immobilized over the coated electrodes.

For microarray applications, DNA strands are not synthesized with a cleavable linker at the 3'-terminus, and the DNA strand should remain immobilized to the surface coating over the electrode where they were assembled. But DNA strands synthesized from the "loose" outer layer of polyalcohol (e.g. polybenzyl alcohol) are cleaved during ethylenediamine (EDA)/ethanol deprotection conditions required for removal of protecting groups on nucleotide bases. As a result, the DNA probes are washed away during deprotection and low fluorescent signals are observed in a hybridization assays with cy-5 labeled complements. We discovered that the "loose layer" can be removed from the electrode coating by "stripping" with hot, concentrated ammonium hydroxide prior to DNA synthesis. The resulting layer after stripping yields a robust, hydroxyl coated film that is suitable for DNA synthesis and stable to final base deprotection with EDA/ethanol. Synthetic probes remain immobilized over the electrodes as evidenced by high fluorescent signals in a cy5 microarray hybridization assay.

In an aspect, embodiments of the present disclosure may include methods of preparing an electrode surface for oligonucleotide synthesis, including (a) cleaning the electrode surface, (b) introducing a diazonium salt to the cleaned electrode surface, (c) electrografting the diazonium salt so as to form a multi-layer film of polyalcohol onto the cleaned electrode surface, in which the multi-layer film may include a loose outer layer of polyalcohol and an inner stable layer of polyalcohol, (d) stripping or removing the loose outer layer of polyalcohol, and (e) performing the oligonucleotide synthesis on the inner stable layer polyalcohol.

In another aspect, the electrode surface may contain platinum, titanium, or a combination thereof.

In another aspect, the electrode surface may be a surface of a semiconductor chip.

In another aspect, the semiconductor chip may be a complementary metal oxide semiconductor (CMOS) chip.

In another aspect, the cleaning in (a) may be performed by using a plasma and/or a compound.

In another aspect, the plasma may be an oxygen plasma only, an Argon plasma only, an oxygen plasma followed by an Argon plasma, or an Argon plasma followed by an oxygen plasma.

In another aspect, the cleaning with the oxygen plasma may be performed from about 1 minute to about 60 minutes, from about 5 minutes to about 55 minutes, from about 10 minutes to about 50 minutes, from about 15 minutes to about 45 minutes, from about 20 minutes to about 40 minutes, from about 25 minutes to about 40 minutes, from about 30 minutes to about 40 minutes, from about 35 minutes to about 40 minutes, from about 36 minutes to about 40 minutes, from about 36 minutes to about 39 minutes, from about 36 minutes to about 38 minutes, or from about 36 minutes to about 37 minutes, and the cleaning with the Argon plasma may be performed from about 1 minute to about 60 minutes, from about 2 minutes to about 55 minutes, from about 3 minutes to about 50 minutes, from about 4 minutes to about 45 minutes, from about 5 minutes to about 40 minutes, from about 6 minutes to about 30 minutes, from about 7 minutes to about 20 minutes, from about 8 minutes to about 15 minutes, from about 9 minutes to about 15 minutes, from about 10 minutes to about 15 minutes, from about 1 minute to about 10 minutes; from about 2 minutes to about 10 minutes, from about 3 minutes to about 10 minutes, from about 4 minutes to about 10 minutes, from about 5 minutes to about 10 minutes, from about 6 minutes to about 10 minutes, from about 7 minutes to about 10 minutes, from about 8 minutes to about 10 minutes, from about 9 minutes to about 10 minutes, or from about 6 minutes to about 7 minutes.

In another aspect, the compound may be piranha solution, acid, base, hydrogen peroxide, or a combination thereof

In another aspect, methods of the present disclosure may further include electrochemically cleaning the plasma-cleaned electrode surface prior to step (b).

In another aspect, the electrochemically cleaning may be performed in the presence of p-toluenesulfonic acid.

In another aspect, the concentration of the diazonium salt may be from about 1 mM to about 1 M, from about 1 mM to about 900 mM, from about 1 mM to about 800 mM, from about 1 mM to about 700 mM, from about 1 mM to about 600 mM, from about 1 mM to about 500 mM, from about 1 mM to about 400 mM, from about 1 mM to about 300 mM, from about 5 mM to about 300 mM, from about 5 mM to about 250 mM, from about 5 mM to about 200 mM, from about 5 mM to about 150 mM, from about 5 mM to about 100 mM, from about 5 mM to about 50 mM, from about 10 mM to about 300 mM, from about 10 mM to about 250 mM, from about 10 mM to about 200 mM, from about 10 mM to about 150 mM, from about 10 mM to about 100 mM, from about 10 mM to about 50 mM, about 5 mM, about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM, or about 100 mM.

In another aspect, the electrografting may be performed in the presence or in the absence of an electrolyte.

In another aspect, the electrolyte may be tetrabutylammonium tetrafluoroborate (TBATFB).

In another aspect, the polyalcohol may be polybenzyl alcohol, polyazobenzene alcohol, or a combination thereof.

In another aspect, the stripping or removing the loose outer layer of polyalcohol may be performed in the presence of ethylenediamine (EDA) and ethanol, ethanol, acetone, dimethylformamide (DMF), ammonium hydroxide, dimethyl sulfoxide (DMSO), methanol, or a combination thereof.

In another aspect, the ratio of EDA:ethanol may be 1:1.

### BRIEF DESCRIPTION OF THE DRAWINGS

FIG. 1 shows structures and synthesis of exemplary diazonium salts used for electrografting on electrodes.
FIG. 2 depicts a method for synthesis of polybenzyl alcohol coated platinum electrodes. Purified diazonium salt from 4-aminobenzyl alcohol is deposited onto the platinum electrode surface by either electrografting or spontaneous grafting mechanisms.

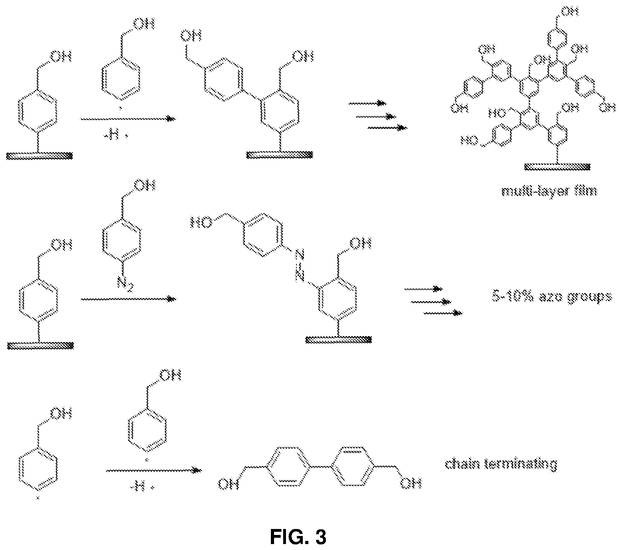

FIG. 3 shows possible compositions of multi-layer films including azo bonds and branching structures. The branched polybenzyl alcohol film gives high yields of cleaved oligonucleotides after electrochemical DNA synthesis.

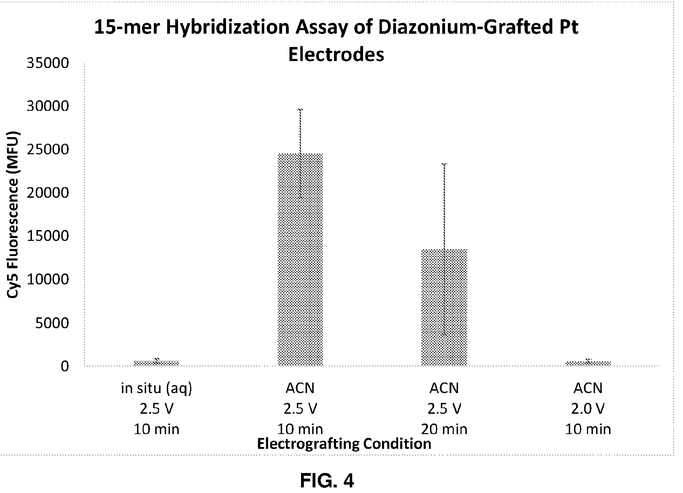

FIG. 4 shows results from DNA synthesis of a 15-mer microarray on a 12,544 electrode chip. A microscope slide sized "chip" was assembled with a hybridization cap to provide a 0.12 mL chamber over the electrode array. The chamber was filled with 60 mM solution of AB-OH diazonium salt in 100 mM tetrabutylammonium tetrafluoroborate electrolyte. Electrografting was performed by applying negative voltage through the Pt electrodes, with ground to surrounding Pt grid. After indicated time and voltage, chips were washed with ACN and used to synthesize a 15-nt oligonucleotide. Chips were hybridized with a 5'-cy5 labelled complementary strand and imaged on a microarray scanner. Fluorescent signal (MFU) were compared *to in situ* (aqueous) method for coating the chip surface.

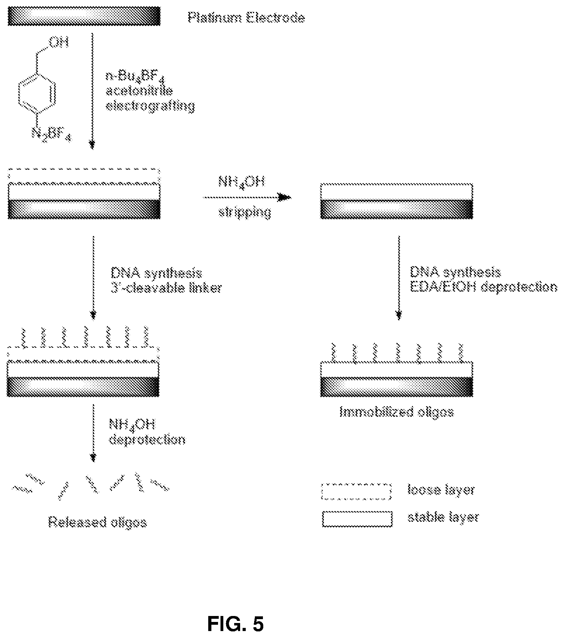

FIG. 5 shows AB-OH Dz coated electrodes with loose or stable layers, and their utility in DNA synthesis applications.
FIG. 6 shows results from DNA synthesis of a 15-mer microarray (94,928 electrode chip). In this experiment, "loose" outer layers of the film were removed with ammonium hydroxide prior to DNA synthesis to yield a stable surface coating with improved immobilization of synthesized DNA probes.
FIG. 7 shows a process for coating electrode array with aryl diazonium for oligonucleotide synthesis in accordance with an embodiment of the present disclosure..

### DETAILED DESCRIPTION

Before the subject disclosure is further described, it is to be understood that the disclosure is not limited to the particular embodiments of the disclosure described below, as variations of the particular embodiments may be made and still fall within the scope of the appended claims. It is also to be understood that the terminology employed is for the purpose of describing particular embodiments and is not intended to be limiting. Instead, the scope of the present disclosure will be established by the appended claims.

### Solid Supports

In an aspect, the support material for oligonucleotide synthesis may comprise a flat (planar) electrode.

The flat electrode may be a dense electrode array comprising a plurality of cells and a surface, where each cell of the plurality of cells includes an anode and a circumferential cathode (grid), where each of the anodes are separately addressable electrodes, and where a thin reaction layer is adsorbed to the surface. This configuration is used to generate acid at the electrode for "detritylation" by oxidation of 1,4-hydroquinone (Maurer, et. al 2006, e34). Current provided to electrodes can be reversed to act as cathodes for reduction of diazonium salts by applying negative DC voltage through an external power supply.

The electrode array devices can be fabricated using standard CMOS technology. This device utilizes alternating array of circular active electrodes and continuous circumferential counter electrodes. In a CMOS process, the semiconductor silicon wafer is fabricated using aluminum wiring and electrodes and then "post-processed" by sputtering another metal, metal alloy, or other conductive material, such as a conductive ceramic. In some embodiments, the metal is a noble metal. In certain embodiments, the electrode is platinum, titanium, or titanium nitride.

Another format is to have a standard electrode array device made with circular electrodes arranged in rows and columns, there are lines separating each "cell" of the electrode array. A cell comprises an electrode and the associated circuitry needed to independently electronically access each electrode individually. In certain embodiments, the wires separating each cell can be raised to the surface of the electrode array (where the electrodes have surface exposure) and function as an array-wide grid of counter electrode for which electrodes are turned on in each electrochemical synthesis step.

The oligonucleotide synthesis may be performed on a support medium comprising a plurality of separately addressable electrodes. In certain embodiments, the electrodes are platinum, titanium, or titanium nitride electrodes.

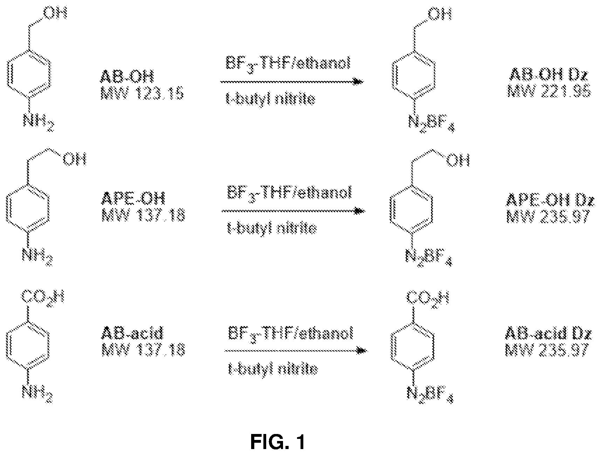

The electrodes can be coated using aryldiazonium salts. Aryldiazonium salts are represented by the generic formula R-Ar-N₂⁺X⁻, where R can be any organic group, such as an alkyl or an aryl, and X is an inorganic or organic anion, such as a halogen, tetrafluoroborate, or tosylate. The term "halogen" represents chlorine, fluorine, bromine, or iodine. Methods for synthesis of aryldiazonium tetrafluoroborate salts are well known. FIG. 1 shows a reproducible method that used ethanol as solvent, boron trifluoride-tetrahydrofuran complex and t-butyl nitrite to diazotize.

In one embodiment, the aryldiazonium salt is a functionalized aryldiazonium salts having the formula:

Y-R-Ar-N₂⁺X⁻,

wherein:
- Y: is a functional group;
- R: is any organic group or is absent; and
- X: is an inorganic or organic anion.

In some embodiments, Ar is selected from phenyl, xylyl, naphthyl, tolyl, and indolyl. In some embodiments, Ar is phenyl.

In some embodiments, Y is -OH, -COOH, or -NH₂. In some embodiments, Y is protected. For example, in some embodiments, Y is protected -OH, protected - COOH, or protected -NH₂.

In some embodiments, R is selected from an alkyl group, an alkenyl group, an aryl group, and a heteroaryl group. In some embodiments, R is selected from a linear or branched C₁-C₁₆ alkyl group, a linear or branched C₁-C₁₂ alkyl group, a linear or branched C₁-C₈ alkyl group. In some embodiments, R is selected from a methyl, ethyl, isopropyl, n-propyl, tert-butyl, isobutyl, n-butyl, n-pentyl, isoamyl and 1,1-dimethylpropyl group.

In some embodiments, X is a halogen, tetrafluoroborate, or tosylate.

In one embodiment, a hydroxyl coating can be applied to an electrode surface (e.g., a platinum electrode surface) using the diazonium salt of 4-aminobenzyl alcohol (AB-OH) and electrochemical reduction (also known as electrodeposition or electrografting).

Although aryldiazonium electrografting onto platinum electrode surfaces has been reported (Bernard, et. al, 2003, Chem. Mater. 3450-3462), the surface coatings did not have suitable functional linker groups for DNA synthesis or attachment of biomolecules using standard chemical coupling methods. The present inventors show here that platinum electrodes can be electrografted with the aryldiazonium salts containing linker groups shown in FIG 1.

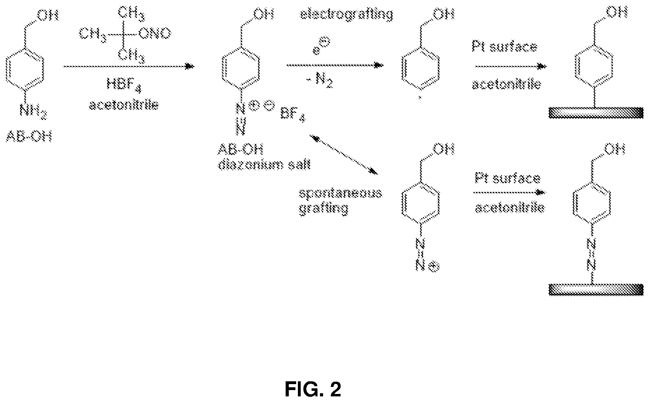

The present inventors have shown that the diazonium salt of AB-OH (AB-OH Dz) reacts with electrical reduction to give a benzyl alcohol coating over the electrode surface (see FIG 2). The benzyl alcohol groups on the surface are reacted with activated phosphoramidites to initiate DNA synthesis. To optimize DNA synthesis performance, the surface can optionally be further modified with long spacer phosphoramidites molecules and cleavable linker molecules prior to addition of the first 3'-nucleoside. CMOS chips with benzyl alcohol coated platinum electrodes are also suitable for electroarray-based assays (Ghindilis, 2007, Biosensors and Bioelectronics, 1853).

The present inventors observed that aryldiazonium salts can also form stable benzyl alcohol films on platinum electrodes by "spontaneous grafting." This process has been reported for coating gold and nickel surfaces by simply dipping the substrates in aqueous or acetonitrile solution of aryldiazonium salts (Mesnage, 2012 Langmuir 11767). Thus, two possible processes and mechanisms for forming the initial metal bond with AB-OH diazonium salts is shown in FIG. 2. In either case, the initial monolayer of aryl groups is further reacted with additional reactive free radical or diazonium monomer groups to give multi-layer films.

Others have shown that electrografted diazonium films on metal surfaces are composed of multiple "layers" including azo bonds and branching structures with variable thickness depending on functional group (Zhang, et. al 2014 Z. Phys. Chem. 557). Thicknesses of 6-7 layers (5 nm) have been reported for coating with some diazonium salts (Bernard, et. al, 2003, Chem. Mater. 3450-3462). Although the structure of the branched polybenzyl alcohol films over platinum electrodes is unclear, the solid surfaces give high yields of oligonucleotides after electrochemical DNA synthesis.

Aryldiazonium salts can be prepared from the starting aniline compound in aqueous conditions (using sodium nitrite) or in anhydrous solvents (using t-butyl nitrite). Reaction of the aniline is rapid, and the aryldiazonium salts are often formed "*in-situ*" without purification. APA-acid diazonium salt obtained by this method was used successfully in aqueous electrografting reactions without purification.

Side products form during synthesis of the aryldiazonium compounds and electrografting is improved by use of purified compounds with removed side products. Although many purified aryldiazonium salts are known (see, for example Colleville et al. (2014) Org Proc R&D, 1128), the present inventors are first to report synthesis of purified 4-(hydroxymethyl)benzenediazonium tetrafluoroborate salt (AB-OH diazonium salt). Purification of aryldiazonium tetrafluoroborate salts is accomplished by precipitation from the reaction mixture or re-precipitation from diethyl ether. No chromatographic methods or crystallization methods have been reported, and their reactivity requires handling at low temperatures. Purified APE-OH diazonium tetrafluoroborate has previously been reported with NMR data (Gam-Derouich, 2010, Surf. Int. Anal. 1050). The present inventors isolated AB-OH and APE-OH diazonium salts in good yield (60-85%) as brown syrups. Aromatic impurities (10-20%) are observed by proton NMR, but the compounds remained stable for months when stored at 0-5 °C. Likewise, purified AB-acid diazonium tetrafluoroborate has been previously reported in the literature (Doyle and Bryker 1978 J.Org. Chem. 1572) and was easily isolated as a pure white solid using the methods described here. When used for electrochemical DNA synthesis, purified AB-OH and APE-OH diazonium salts have the advantage of providing a hydroxyl coating over the electrode without need for further functionalization. The stable tetrafluoroborate salts are easily stored to facilitate manufacturing chips and provide added control in coating the electrodes.

### Oligonucleotide synthesis

Standard synthetic methods for oligonucleotides are known in the art (e.g., U.S. Pat. Nos. 5,750,666, 6,111,086, 6,008,400, and 5,889,136).

Support bound oligonucleotide synthesis relies on sequential addition of nucleotides to one end of a growing chain. Typically, a first nucleoside (having protecting groups on any exocyclic amine functionalities present) is attached to the solid support medium and activated phosphite compounds (which also bear appropriate protecting groups) are added stepwise to elongate the growing oligonucleotide. Additional methods for solid-phase synthesis may be found in Caruthers U.S. Pat. Nos. 4,415,732; 4,458,066; 4,500,707; 4,668,777; 4,973,679; and 5,132,418; and Koster U.S. Pat. Nos. 4,725,677).

Electrochemical reagents capable of electrochemically removing protecting groups from chemical functional groups on the molecule are generated at selected electrodes by applying a sufficient electrical potential to the selected electrodes. Removal of a protecting group, or "deprotection," in accordance with the invention, occurs at selected molecules when a chemical reagent generated by the electrode acts to deprotect or remove, for example, an acid or base labile protecting group from the selected molecules.

In one example of the present disclosure, a terminal end of a monomer nucleotide, or linker molecule (i.e., a molecule which "links," for example, a monomer or nucleotide to a substrate) is provided in accordance with the present disclosure, which is protected with a protecting group removable by an electrochemically generated reagent. The protecting group(s) is exposed to reagents electrochemically generated at the electrode and removed from the monomer, nucleotide or linker molecule in a first selected region to expose a reactive functional group. The substrate is then contacted with a first monomer or pre-formed molecule, which bonds with the exposed functional group(s). This first monomer or pre-formed molecule may also bear at least one protected chemical functional group removable by an electrochemically generated reagent.

The term "protecting group" (or "blocking group)" as used herein, refers to a labile chemical moiety which is known in the art to protect a hydroxyl, amino or thiol group against undesired reactions during synthetic procedures. Protecting groups as known in the art are described generally in T. H. Greene and P. G. M. Wuts, 1999, Protective Groups in Organic Synthesis, 3rd edition, John Wiley & Sons, New York. Examples of hydroxyl protecting groups include, but are not limited to, benzyloxycarbonyl, 4-nitrobenzyloxycarbonyl, 4-bromobenzyloxycarbonyl, 4-methoxybenzyloxycarbonyl, methoxycarbonyl, tert-butoxycarbonyl (BOC), isopropoxycarbonyl, diphenylmethoxycarbonyl, 2,2,2-trichloroethoxycarbonyl, 2-(trimethylsilyl)ethoxycarbonyl, 2-furfuryloxycarbonyl, allyloxycarbonyl (Alloc), acetyl (Ac), formyl, chloroacetyl, trifluoroacetyl, methoxyacetyl, phenoxyacetyl, benzoyl (Bz), methyl, t-butyl, 2,2,2-trichloroethyl, 2-trimethylsilyl ethyl, 1,1-dimethyl-2-propenyl, 3-methyl-3-butenyl, allyl, benzyl (Bn), para-methoxybenzyldiphenylmethyl, triphenylmethyl (trityl), 4,4'-dimethoxytriphenylmethyl (DMT), substituted or unsubstituted 9-(9-phenyl)xanthenyl (pixyl), tetrahydrofuryl, methoxymethyl, methylthiomethyl, benzyloxymethyl, 2,2,2-trichloroethoxymethyl, 2-(trimethylsilyl)ethoxymethyl, methanesulfonyl, para-toluenesulfonyl, trimethylsilyl, triethylsilyl, and triisopropylsilyl. In some embodiments, the protecting group is DMT.

In some embodiments, the hydroxyl protecting group is a silyl protecting group. Examples of silyl protecting groups include, but are not limited to, 2-(trimethylsilyl)ethoxycarbonyl, 2-trimethylsilyl ethyl, 2-(trimethylsilyl)ethoxymethyl, trimethylsilyl (TMS), triethylsilyl (TES), triisopropylsilyl (TIPS), isopropyldimethylsilyl (IPDMS), diethylisopropylsilyl (DEIPS), t-butyldimethylsilyl (TBS), t-butyldiphenylsilyl (TBDPS), tetraisopropyldisiloxanylidene (TIPDS), di-t-butylsilylene (DTBS), and t-butyldimethylsilyl (TBDMS).

The monomers or pre-formed molecules can then be deprotected in the same manner to yield a second set of reactive chemical functional groups. A second monomer or pre-formed molecule, which may also bear at least one protecting group removable by an electrochemically generated reagent, is subsequently brought into contact with the substrate to bond with the second set of exposed functional groups. Any unreacted functional groups can optionally be capped at any point during the synthesis process. The deprotection and bonding steps can be repeated sequentially at this site on the substrate until polymers or oligonucleotides of a desired sequence and length are obtained.

The substrate having one or more molecules bearing at least one protected chemical functional group bonded thereto may be proximate an array of electrodes, which array is in contact with a buffering or scavenging solution. Following application of an electric potential to selected electrodes in the array sufficient to generate electrochemical reagents capable of deprotecting the protected chemical functional groups, molecules proximate to the selected electrodes are deprotected to expose reactive functional groups, thereby preparing them for bonding. A monomer solution or a solution of pre-formed molecules, such as proteins, nucleic acids, polysaccharides, and porphyrins, is then contacted with the substrate surface and the monomers or pre-formed molecules bond with the deprotected chemical functional groups.

The methods described herein may further comprise reacting said monomer-functionalized support medium with a capping agent; and optionally treating said monomer-functionalized support medium with an oxidizing agent.

Following oligonucleotide synthesis, the oligonucleotides can be released from or immobilized onto the solid support medium. These methods may comprise a step of treating the oligonucleotide with a reagent effective to cleave the oligonucleotide from the support medium, preferably from the linker attached to the support medium. In some such embodiments, the treating of the oligonucleotide with a reagent effective to cleave the oligonucleotide removes protecting groups present on the oligonucleotide. In some embodiments, the cleaved oligonucleotide has a 3' unmodified terminal hydroxyl group at the site of cleavage. In various embodiments, solid support medium is treated with anhydrous ammonia for a period of time sufficient to cleave the oligonucleotide.

The cleaved oligonucleotide may then be prepared by procedures known in the art, for example by size exclusion chromatography, high performance liquid chromatography (e.g. reverse-phase HPLC), differential precipitation, etc. In some embodiments according to the present invention, the oligonucleotide is cleaved from a solid support medium while the 5'-OH protecting group is still on the ultimate nucleoside. This so-called DMT-on (or trityl-on) oligonucleotide is then subjected to chromatography, after which the DMT group is removed by treatment in an organic acid, after which the oligonucleotide is de-salted and further purified to form a final product.

### Use of oligonucleotides

The methods and compositions provided herein are useful for genome editing libraries such as CRISPR gRNA screening libraries and shRNA screening libraries, targeted sequencing such as hybrid-capture or MIP, mutagenesis libraries, generation of oligos for *in situ* hybridization applications, and generation of pools of oligos for DNA data storage.

### Current sourcing

The term "current sourcing" used herein refers to maintaining a constant current by adjusting the supplied voltage to achieve the desired current and application of a constant current to an electrolytic cell.

### Voltage sourcing

The term "voltage sourcing" used herein refers to setting a constant voltage in an electrolytic cell independent of output current and application of a constant voltage to an electrochemical cell.

### Electrolytic cell

The term "electrolytic cell" used herein refers to electrochemical cells that may require external sources of electrical energy (voltage applied between two electrodes) to drive a chemical reaction that would not otherwise occur. Electrochemical cells may be devices capable of either generating electrical energy from chemical reactions or using electrical energy to cause chemical reactions. Electrochemical cells that use electrical energy to generate chemical reactions, via electrolysis, for example, are called electrolytic cells.

### Multi-layer film of polyalcohol

The term "multi-layer film of polyalcohol" used herein refers to possible side reactions that may occur in electrografting (and spontaneous grafting) processes. Upon electrografting of functionalized aryldiazonium salts by contacting with (spontaneous) or energizing solid support, free radicals of diazonium monomers can react with surface to form a monolayer coating. This initial monolayer coating may be further reacted with additional free radical of the diazonium to form a multi-layer that can contain polyalcohol (e.g. polybenzyl alcohol). Further, possible compositions of multi-layer films can include azo bonds and branching structures.

### Loose outer layer of polyalcohol

The term "loose outer layer of polyalcohol" used herein refers to layer of multi-layer film of polyalcohol, which may be not stable to oligonucleotide synthesis processes, and which can be removed with chemical treatments.

### Examples

### EXAMPLE 1

Synthesis of aryldiazonium tetrafluoroborate salts. The salts were prepared using variations of literature methods. Solvents were removed with a rotary evaporator and further drying for several hours on a high vacuum pump (<1 mm Hg). Proton NMR spectra were obtained on a 500 MHz Bruker instrument.

Diazonium salts of AB-acid, APE-OH and AB-OH shown in FIG.1 were prepared by dissolving the starting aniline in ethanol and adding 1.5 equivalents of boron trifluoride-THF complex. The solution was cooled and 1.2 equivalents of t-butylnitrite were added (see, for example Colleville et al. (2014) Org Proc R&D, 1128). After warming to room temperature, the salts were collected directly (AB-acid) or as dark brown oils (APE-OH and AB-OH) and further precipitated from diethyl ether. AB-acid diazonium tetrafluoroborate salt was isolated in 45% yield as a white solid, and proton NMR was consistent with literature values (Doyle et al. (1979) J. Org. Chem., 1572) but increased field strength showed aromatic protons as doublets of triplets instead of reported doublets. APE-OH and AB-OH were isolated as dark orange, syrups that were soluble in both acetonitrile and water. Proton NMR spectra of the aryldiazonium salts showed excellent purity when dissolved in CD₃CN, but slowly decomposed to another compound (possibly the bisphenyl derivative, ~50% after 1 week at room temperature). Decomposition was more rapid in d6-DMSO.

**4-(2-hydroxyethyl)benzenediazonium tetrafluoroborate (APE-OH Dz).** To a stirred solution of 0.5 g (3.64 mmole) of 2-(4-aminophenyl)ethanol in 8.6 mL of ethanol was added 0.6 mL of BF₃·THF complex (5.46 mmole). The solution was cooled in an ice bath under argon, and 0.58 mL (4.9 mmol) of t-butylnitrite was added. The solution was allowed to warm to room temperature and stirred for 1 hour. A dense brown oil precipitated that was collected with a pipette. 20 mL of diethyl ether was added and more oil precipitated. The combined oil was washed by shaking with 20 mL of diethyl ether, and the remaining orange syrup was dried under vacuum to give 0.46 g (53% yield) of APE-OH diazonium salt. ¹H NMR (d6-DMSO): δ, ppm 8.58, d, 2H, J=8.7 Hz; 7.84, d, 2H, J=8.7 Hz; 4.38, br s, 1H; 3.72, t, 2H, J=6 Hz; 2.98, t, 2H, J=6 Hz.

**4-(hydroxymethyl)benzenediazonium tetrafluoroborate (AB-OH Dz).** To a stirred solution of 0.45 g (3.64 mmole) of (4-aminophenyl)methanol in 9 mL of ethanol was added 0.6 mL of BF₃·THF complex (5.46 mmole). The solution was cooled in an ice bath under argon, and 0.58 mL (4.9 mmol) of t-butylnitrite was added. After 30 min the solution was allowed to warm to room temperature and stirred for 1 hour. TLC (10% methanol/ethyl acetate) showed no remaining aniline (Rf = 0.63, yellow stain with p-anisaldehyde), with a brown spot at the baseline. The dense brown oil precipitate was collected with a pipette. 10 mL of diethyl ether was added and more oil precipitated. The combined oil was washed by shaking with 2x10 mL of diethyl ether, and the remaining orange syrup was dissolved in acetonitrile and rotovapped to dryness to give 0.69 g (85% yield) of AB-OH diazonium salt. ¹H NMR (CD₃CN): δ, ppm 8.46, d, 2H, J=9 Hz; 7.91, d, 2H, J=9 Hz; 4.88, s, 2H.

**4-carboxybenzenediazonium tetrafluoroborate (AB-acid Dz).** To an ice cold solution of 0.5 g (3.64 mmole) of 4-aminobenzoic acid in 9 mL of ethanol was added 0.6 mL of BF₃·THF complex (5.46 mmole) and 0.58 mL of t-butylnitrite was added, and after 30 min the solution was allowed to warm to room temperature and stirred for 1 hour. The white precipitate was collected by filtration, washed with 3x3 mL of ethanol, and dried under vacuum to give 0.39 g (45% yield) of AB-acid diazonium salt. ¹H NMR (CD₃CN): δ, ppm 8.62, d of t, 2H, J=9 Hz, 2 Hz; 8.46, d of t, 2H, J=9 Hz, 2 Hz.

### EXAMPLE 2

Synthesis of benzyl alcohol coated chips (12k array). CMOS chips with array of 12,544 platinum electrodes were obtained from CustomArray. The chips were cleaned with oxygen plasma (36 minutes) and stored in a desiccator prior to use. As shown in FIG. 2, a solution of 13.3 mg (60 micromoles) of AB-OH Dz was dissolved in 1 mL of electrolyte (0.1 M tetrabutylammonium tetrafluoroborate in anhydrous acetonitrile). A plasma cleaned chip was inserted into the hybridization chamber of an ElectraSense^{™} Reader (see Ghindilis et al., Biosensors and Bioelectronics 22 (2007) 1853-1860). The chamber (0.12 mL) was filled with the (orange) AB-OH Dz solution and the electrodes were energized at -2.5 volts for 10 minutes. The (darker orange) diazonium salt solution was removed with a pipettor, and the chip was washed with 5 x 1 mL of acetonitrile. The dried chip was stored in a desiccator prior to DNA synthesis.

A microscope slide sized "chip" was assembled with a hybridization cap to provide a 0.12 mL chamber over the electrode array. The chamber was filled with 60 mM solution of AB-OH diazonium salt in 100 mM tetrabutylammonium tetrafluoroborate electrolyte. Electrografting was performed by applying negative voltage through the Pt electrodes, with ground to surrounding Pt grid. After indicated time and voltage, chips were washed with ACN and used to synthesize a 15-nt oligonucleotide. Chips were hybridized with a 5'-cy5 labelled complementary strand and imaged on a microarray scanner. Fluorescent signal (MFU) were compared *to in situ* (aqueous) method for coating the chip surface. Results of immobilized DNA synthesis is shown in FIG. 4.

### EXAMPLE 3

Synthesis of benzyl alcohol coated chips (Electrografted, 90k array). CMOS chips with array of 94,928 platinum electrodes were obtained from CustomArray. A 90k prototype of the ElectraSense Reader was used to repeat the 12k array coating process described above. Initially, DNA synthesis yields were very low. Synthesis yields were improved by electrochemically cleaning the chips with 0.1 M p-toluenesulfonic acid in acetonitrile (10 namp/electrode, 1 min) and rinsing with 5x1 mL of acetonitrile. Electrografting with 60 mM AB-OH Dz used -1.5 volts for 20 minutes. The diazonium salt solution was removed and the chip was washed with 5 x 1 mL of acetonitrile. The dried chip was stored in a desiccator prior to DNA synthesis. DNA synthesis results with the acetonitrile washed 90k chips showed large amount of 530 nm autofluorescence, a phenomenon associated with successful electrochemical DNA synthesis (unpublished results). DNA yields of released 120-mer oligos were further measured by qPCR as shown in Table 1.

**Table 1.**

| **Condition** | **Chamber** | **Conc (ng/µL)** | **Ct** |
|---|---|---|---|
| Su-coated Unstripped | 0 | 64.2 | 24.1 |
| Diazonium-coated Unstripped | 1 | 67.7 | 22.5 |
| Su-coated NH₄OH Strip | 2 | 40.8 | 26.6 |
| Diazonium-coated NH₄OH strip | 3 | 29.2 | 27.1 |

The data in Table 1 shows that AB-OH Dz coated electrodes (94,928 electrode chip) gives good DNA synthesis yield of 120-mer oligonucleotide pools. Yield is compared to previously described sucrose coated electrodes using either 260 nm absorbance (Nanodrop) or qPCR assays.

### EXAMPLE 4

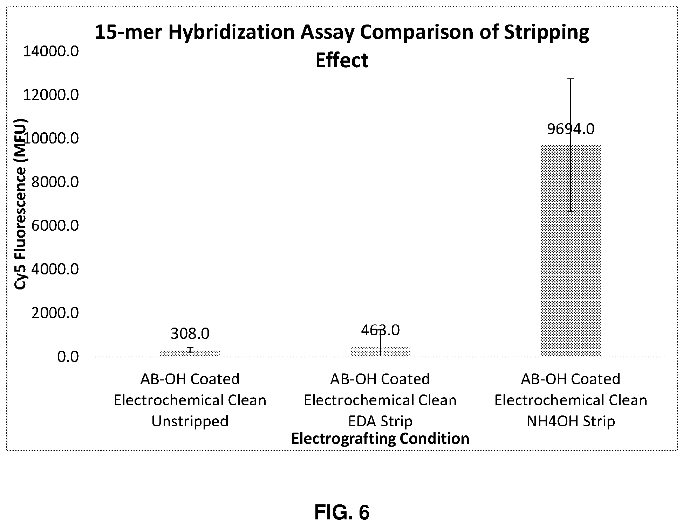

Synthesis of "stripped" benzyl alcohol AB-OH coated chips (90k array). Synthetic DNA strands are cleaved from the AB-OH coated DNA chip during DNA deprotection with EDA/ethanol. This is presumably due to DNA synthesis from a "loose" layer of hydroxyl coated structures on the coated electrode surface. To improve amount of immobilized DNA, the "loose" layer of film is stripped off by treating the AB-OH coated 90k chip described above with concentrated ammonium hydroxide (overnight, 65 degrees Celsius) in a 0.8 mL stripping chamber (O-ring seal). Further washing with acetonitrile gave "stripped" AB-OH coated electrodes ready for electrochemical DNA synthesis. Fluorescent microarray analysis showed 10x increased signal with NH₄OH stripped chips as shown in FIG. 6.

### EXAMPLE 5

Spontaneous grafting process for benzyl alcohol coated chips (90k arrays). The process described in EXAMPLE 3 was repeated in the 90 prototype ElectraSense Reader. The chips were assembled in the 0.16 mL chamber and electrochemically cleaned with 0.1 M p-toluenesulfonic acid in acetonitrile (10 namp/electrode, 1 min) and rinsing with 5x1 mL of acetonitrile. The diazonium salt solution (60 micromoles AB-OH Dz in 1 mL of 0.1 M tetrabutylammonium tetrafluoroborate in anhydrous acetonitrile) was added and the chip was kept at room temperature without energizing the electrodes. After washing with 5 x 1 mL of acetonitrile, the dried chip was stripped with concentrated ammonia as usual (65 °C overnight). Spontaneously grafted chips showed similar performance to electrografted chips with large amount of 530 nm autofluorescence prior to deprotection with EDA/ethanol. Resulting arrays had 25% lower signal than electrografted arrays using fluorescent microarray analysis.

### EXAMPLE 6

Electrochemical DNA Synthesis of 15-mer Microarrays. In this example, either CombiMatrix CustomArray^{™} 12k or 90k microarray DNA synthesizers were used to assemble 15-mer oligonucleotides from polybenzyl alcohol coated chips. Each chip was assembled into a thin (0.16 mL) flow through chamber that allows DNA synthesis reagents to be pumped in and out while each electrode is electronically addressed via computer control. The electrochemical synthesis used phosphoramidite chemistry coupled with electrochemical deblocking of the dimethoxytrityl protecting groups at each coupling step. Electrochemical deblocking involved turning on an electrode to generate acidic conditions at the electrode that were sufficient to remove the protecting group only at the active electrode. Buffer in the solution used for electrochemical deblocking and natural diffusion prevented deblocking at non-activated electrodes (Maurer, et al. 2006 PLoS One e34). Removal of the dimethoxytrityl protecting group allowed addition of the next phosphoramidite. Before synthesis of the designed 15-mer DNA sequence, a 10-dT spacer was added directly to each AB-OH coated electrodes. The dT₁₀ spacer gave improved fluorescent signal in microarray analysis for both 12k and 90k formats. Then electrodes were selectively detritylated as required to react with the next added nucleoside phosphoramidite. After DNA synthesis was complete, the chips were deprotected with 1:1 / ethylenediamine:ethanol (4 hr, 65 °C) in a 0.8 mL stripping chamber (O-ring seal). After cooling in ice, the chip was removed, washed with acetonitrile, and stored dry for fluorescent microarray analysis.

### EXAMPLE 7

Fluorescent microarray analysis. Synthetic microarrays were designed with identical 15-mer DNA sequences immobilized at each electrode. A reverse complementary sequence was obtained with a cy5 fluorescent dye at the 5'-terminus. A hybridization solution containing the cy5 oligonucleotide was prepared in a 0.12 mL hybridization chamber over the electrodes. After at least 1 hour at 37 °C, the hybridization solution was removed and the chip washed several times with buffer. A lifter slip was inserted over the wet electrodes and the assembly was inserted into a Molecular Devices GenePix 4000 or 4400 fluorescence scanner. Each chip was optimized for highest signal by adjusting the gain and focal length during imaging. Images of the fluorescent electrodes were processed with custom software to measure fluorescent signal at each electrode in the array. The images showed uniform distribution of spots and uniform density of fluorescence across the electrodes. Average fluorescence was measured and shown with standard deviations in FIG.4 (12k electrode format) and FIG. 6 (90k electrode format). AB-OH coated electrodes with both arrays showed mean fluorescence units of ~25,000 counts.

### EXAMPLE 8

Electrochemical DNA Synthesis of 120-mer Pools. In this example a CombiMatrix CustomArray^{™} 90k format microarray DNA synthesizer was used to assemble pools of 120-mer oligonucleotides from polybenzyl alcohol coated chips. 90k chips were inserted into the 0.16 mL flow through synthesis chamber and washed with anhydrous acetonitrile immediately before use. For this study, a base cleavable linker phosphoramidite was manually added to the AB-OH Dz coated electrode surfaces. Chemical Phosphorylation Reagent (CPR, Glen Research, Sterling VA) was dissolved in acetonitrile to give a 0.1 M solution, activated with equal volume of 4,5-dicyanoimidazole (0.25 M DCI in acetonitrile), then immediately injected over the polybenzyl alcohol coated electrodes. After 5 min, excess CPR was removed and unreacted benzyl alcohol groups on the film surface were capped as acetate esters. After electrochemical detritylation of the oxidized CPR, the remaining nucleotide phosphoramidites for each 120-mer sequence were assembly as described above. Finally the 5-terminal DMT was removed from all sequences and the chips were deprotected in concentrated ammonia (65 °C, overnight). CPR hydrolyzes to release the 120-mer oligonucleotides from the coated electrodes with 3'-phosphate modifications. The ammonia solution is dried on a SpeedVac (60 °C, 2 h, 35 mm Hg). Each pool is purified by gel filtration column in a final concentration of 0.1 mL. Yield of full length product was determined by qPCR analysis as shown in Table 1.

### EXAMPLE 9

qPCR analysis of pooled 120-mers (90k). Synthetic pools of oligonucleotides were of variable sequence, but 120-mer control sequences (50 electrodes per chip) were prepared as internal controls. These sequences were designed with PCR primer binding sites to allow detection by quantitative PCR as shown in Table 1.

### EXAMPLE 10

**Table 2. Exemplary method for coating electrode array with aryl diazonium**

| Cleaning/preparation of electrode surface | | |
|---|---|---|
| Plasma cleaning | 36 minutes of Oxygen plasma followed by 6 minutes of Argon plasma | Notes: |
| | | The oxygen plasma cleaning step may be used to chemically react Oxygen plasma with any organic contaminants on the surface of the electrode. |
| | | The Argon plasma may be a physical etch where ionized Argon may physically bombard the surface to remove the top level of substrate (CMOS chip) surface atoms. This Argon step can ensure that Platinum electrode may be free of adsorbed contaminants |
| | | Other plasma cleaning protocols may also be used, e.g., Oxygen only protocols, Argon only protocols, varying time of each from 1 minute to 60 minutes. |
| | | The intensity of these plasma cleaning steps may be dictated by the input power. |
| Electrochemical | After plasma cleaning, the | Notes: |
| cleaning | CMOS chips were stored in a desiccator until coating with diazonium. | |
| | | Although electrochemical cleaning may be optional for diazonium coating to work, oligonucleotide synthesis can be enhanced using chips that have been treated using electrochemical cleaning protocol. |
| | Immediately preceding diazonium coating, the chips are electrochemically cleaned to ensure a contaminant free surface. | |
| | | Many variations of electrochemical cleaning were tested, which may include, generally, current sourcing or voltage sourcing the electrodes in a solution with an electrolyte. |
| | To electrochemically clean, chips were put into a sealed chamber that may allow both fluidic delivery and electronic control of chips. To this chamber, a solution of 0.1M solution of p-toluenesulfonic acid in Acetonitrile (ACN) was delivered to submerge the electrode array. | |
| | | Also the time and number of sourcing events can vary from just 1 to as many as 120 pulses for varying time/pulse. |
| | | Also tested were many different electrolyte solutions, both aqueous and organic solutions. Typically, electrochemically cleaning was performed in H₂SO₄ solution in an aqueous system and p-toluenesulfonic acid in an organic solvent, such as ACN. |
| | Electrochemical cleaning: | |
| | All electrodes in the array were addresses and set to current source +10nA per electrode for 10 seconds. | |
| | Immediately following this, all electrodes were | |
| | grounded and the common electrode ("grid" circumferential electrode) surrounding these electrodes was set to current source +10nA per the total number of electrodes grounded. This cycle was repeated a total of 6 times. Thus, the cleaning method pulsed the electrode by current sourcing +10nA for 10 second, then -10nA for 10 seconds for 6 total cycles. | |

| Electrografting diazonium salt | | |
|---|---|---|
| Diazonium coating | Diazonium coating was performed immediately following electrochemical cleaning in the same fluidic/electronic control chamber. | Notes: |
| | | Aryldizonium salts used in electrode functionalization may include: 4-(hydroxymethyl)benzenediazonium tetrafluoroborate salt (AB-OH diazonium salt), 4-(2-hydroxyethyl)benzenediazonium tetrafluoroborate salt (APE-OH diazonium salt), and 4-carboxybenzenediazonium tetrafluoroborate salt (AB-acid diazonium salt). |
| | After electrochemical cleaning, chips were washed a number of times with acetonitrile. Then, a solution of AB-OH diazonium, for example, was introduced into the chamber. The diazonium | |
| | solution consists of 60 mM AB-OH diazonium salt in acetonitrile with 0.1 M Tetrabutylammonium tetrafluoroborate (TBATFB) as the supporting electrolyte. | The concentration of AB-OH diazonium salt varied from 10 mM to 200 mM, preferably about 60 mM, in Acetonitrile. |
| | | The supporting electrolyte TBATFB has been tested at 0 mM to 1 M. A supporting electrolyte may be not necessary since the AB-OH diazonium salt can act itself as an electrolyte in the solution. |
| | All electrodes in the array that were to be coated (typically all electrodes) were set to -2.0 V for 2 minutes to drive the electrografting. | |
| | | Other quaternary ammonium salts, e.g., Tetramethylammonium tetrafluoroborate, Tetraethylammonium tetrafluoroborate, Tetrabutylammonium hexafluorophosphate, and Tetraethylammonium p-toluenesulfonate, can also be used as supporting electrolytes. |
| | After electrografting, the chips were washed with acetonitrile 3 times. | |

| Post electrografting stripping | | |
|---|---|---|
| Stripping loose outer layer of polybenzyl alcohol (or polyazobenzene | In the same chamber the chips were incubated with methanol for 5 minutes, washed with methanol, then acetonitrile. | Notes: |
| | | Although stripping the loose outer layer of polybenzyl alcohol (or polyazobenzene alcohol) may be |
| alcohol) | | performed using ammonium hydroxide solution, this process may be cumbersome, however. |
| | Chips were then dried with argon and can be stored or used immediately for oligonucleotide synthesis. | |
| | | By simply washing/incubating with DMSO or Methanol, it was enough to remove the loose outer layer. |
| | | Stripping off the loose outer layer made the oligo synthesis results more reproducible than that without stripping. |
| | | Without stripping, the loose outer layer can still be used for oligo synthesis as the loose outer layer may be to some extent labile but somewhat stable to the solutions used in oligonucleotide synthesis (ACN, THF). |

Table 2 summarizes method for coating electrode array with aryl diazonium for oligonucleotide synthesis.

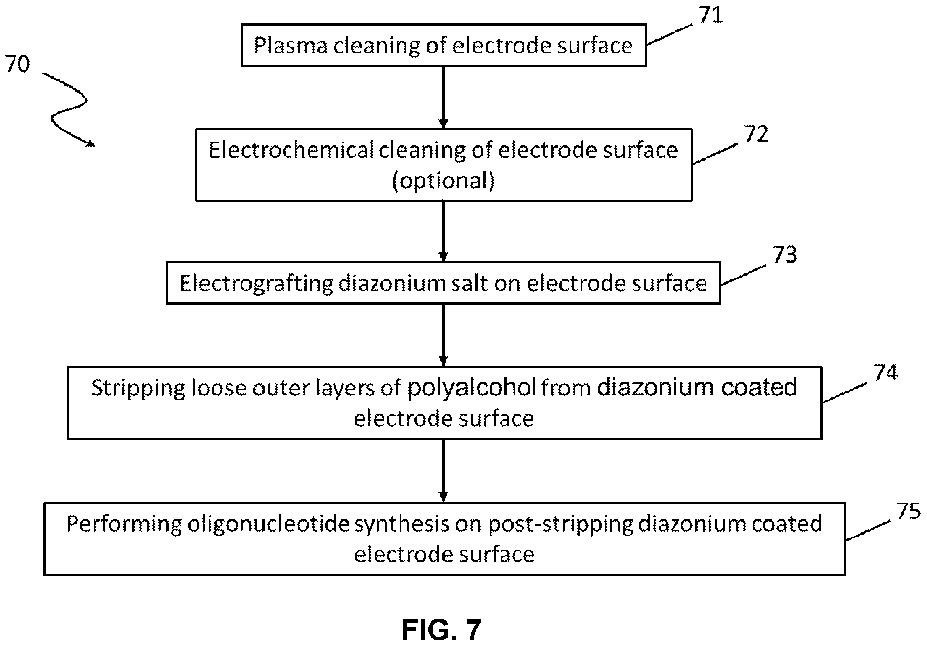

FIG. 7 shows process (70) for coating electrode array with aryl diazonium for oligonucleotide synthesis. Surface of oligonucleotide synthesis chips, such as CMOS chips, may be cleaned by plasma cleaning (71), e.g., 36 minutes of oxygen plasma followed by 6 minutes of Argon plasma. Optionally, immediately preceding diazonium coating, plasma-cleaned surface may be further electrochemically cleaned (72), e.g., by pulsing electrodes by current sourcing +10nA for 10 second, then -10nA for 10 seconds for 6 total cycles in H₂SO₄ solution in an aqueous system and p-toluenesulfonic acid in an organic solvent, e.g., ACN, to ensure a contaminant free surface. Immediately following plasma cleaning or following plasma cleaning and electrochemical cleaning, diazonium coating is performed by electrografting diazonium salts on electrochemically cleaned surface (73), e.g., -2.0 V for 2 minutes to drive the electrografting. After electrografting, stripping loose outer layers of polyalcohol, e.g., polybenzyl alcohol and/or polyazobenzene alcohol, from diazonium coated surface (74), e.g., using DMSO or methanol. Performing oligonucleotide synthesis on post-stripping diazonium coated electrode surface (75).

## Claims

1. A solid support system for synthesis of oligonucleotides, wherein the support is an electrode coated with a thin film comprising polyalcohol,
wherein the electrode is prepared by a method comprising
(a) cleaning the electrode surface,
(b) introducing a diazonium salt to the cleaned electrode surface,
(c) electrografting the diazonium salt so as to form a multi-layer film of polyalcohol onto the cleaned electrode surface,
wherein the multi-layer film comprises a loose outer layer of the polyalcohol and an inner stable layer of the polyalcohol,
(d) stripping the loose outer layer of the polyalcohol, and
(e) obtaining the inner stable layer of the polyalcohol for the synthesis of oligonucleotides.

2. The solid support system of claim 1, wherein the thin film is formed by electrografting of functionalized aryldiazonium salts having the formula:
Y-R-Ar-N₂⁺X⁻,
wherein:
Ar is selected from phenyl, xylyl, naphthyl, tolyl, and indolyl
Y is -OH, -COOH, or -NH₂optionally protected;
R is selected from an alkyl group, an alkenyl group, an aryl group, and a heteroaryl group; and
X is a halogen, tetrafluoroborate, or tosylate.

3. A method of preparing an electrode surface for oligonucleotide synthesis, comprising
(a) cleaning the electrode surface,
(b) introducing a diazonium salt to the cleaned electrode surface,
(c) electrografting the diazonium salt so as to form a multi-layer film of polyalcohol onto the cleaned electrode surface,
wherein the multi-layer film comprises a loose outer layer of polyalcohol and an inner stable layer of polyalcohol,
(d) stripping the loose outer layer of polyalcohol, and
(e) performing the oligonucleotide synthesis on the inner stable layer of polyalcohol.

4. The method of claim 3, wherein the electrode surface comprises platinum, titanium, or a combination thereof.

5. The method of claim 3 or 4, wherein the electrode surface is a surface of a semiconductor chip.

6. The method of claim 5, wherein the semiconductor chip is a complementary metal oxide semiconductor (CMOS) chip.

7. The method of any one of claims 3-6, wherein the cleaning in (a) is performed by using a plasma and/or a compound.

8. The method of claim 7, wherein the plasma is an oxygen plasma only, an Argon plasma only, an oxygen plasma followed by an Argon plasma, or an Argon plasma followed by an oxygen plasma.

9. The method of any one of claims 7-8, wherein the compound is a piranha solution, an acid, a base, hydrogen peroxide, or a combination thereof.

10. The method of claim 3, further comprising electrochemically cleaning the plasma-cleaned electrode surface prior to step (b).

11. The method of claim 10, wherein the electrochemically cleaning is performed in the presence of p-toluenesulfonic acid.

12. The method of any one of claims 3-11, wherein the diazonium salt comprises the formula:
Y-R-Ar-N₂⁺X⁻,
wherein:
Ar is selected from phenyl, xylyl, naphthyl, tolyl, and indolyl;
Y is -OH, -COOH, or -NH₂, optionally protected;
R is selected from an alkyl group, an alkenyl group, an aryl group, and a heteroaryl group; and
X is a halogen, tetrafluoroborate, or tosylate.

13. The method of claim 12, wherein the diazonium salt is selected from the group consisting of 4-(hydroxymethyl)benzenediazonium tetrafluoroborate salt (AB-OH diazonium salt), 4-(2-hydroxyethyl)benzenediazonium tetrafluoroborate salt (APE-OH diazonium salt), and 4-carboxybenzenediazonium tetrafluoroborate salt (AB-acid diazonium salt).

14. The method of any one of claims 3-13, wherein the polyalcohol is polybenzyl alcohol, polyazobenzene alcohol, or a combination thereof.

15. The method of any one of claims 3-14, wherein the stripping the loose outer layer of polyalcohol is performed in the presence of ethylenediamine (EDA) and ethanol, ethanol, acetone, dimethylformamide (DMF), ammonium hydroxide, dimethyl sulfoxide (DMSO), methanol, or a combination thereof.

## Patentansprüche

1. Ein festes Trägersystem zur Synthese von Oligonukleotiden, wobei der Träger eine Elektrode ist, die mit einem dünnen Film beschichtet ist, der einen Polyalkohol aufweist,
wobei die Elektrode durch ein Verfahren hergestellt wird, das Folgendes aufweist:
(a) Reinigen der Elektrodenoberfläche,
(b) Aufbringen eines Diazoniumsalzes auf die gereinigte Elektrodenoberfläche,
(c) Elektrografting des Diazoniumsalzes, um einen mehrschichtigen Film aus Polyalkohol auf der gereinigten Elektrodenoberfläche zu bilden,
wobei der mehrschichtige Film eine lockere äußere Schicht des Polyalkohols und eine innere stabile Schicht des Polyalkohols aufweist,
(d) Abtragen der losen äußeren Schicht des Polyalkohols und
(e) Erhalten der inneren stabilen Schicht des Polyalkohols zur Synthese von Oligonukleotiden.

2. Das feste Trägersystem nach Anspruch 1, wobei der dünne Film durch Elektrografting von funktionalisierten Aryldiazoniumsalzen mit der Formel:
Y-R-Ar-N₂⁺X⁻,
gebildet wird, wobei:
Ar aus Phenyl, Xylyl, Naphthyl, Tolyl und Indolyl ausgewählt ist
Y -OH, -COOH oder -NH2 ist, optinal geschützt;
R aus einer Alkylgruppe, einer Alkenylgruppe, einer Arylgruppe und einer Heteroarylgruppe ausgewählt ist; und
X ein Halogen, Tetrafluorborat oder Tosylat ist.

3. Verfahren zur Herstellung einer Elektrodenoberfläche für die Oligonukleotidsynthese, das eine Elektrodenoberfläche aufweist
(a) Reinigen der Elektrodenoberfläche,
(b) Aufbringen eines Diazoniumsalzes auf die gereinigte Elektrodenoberfläche,
(c) Elektrografting des Diazoniumsalzes, um einen mehrschichtigen Film aus Polyalkohol auf der gereinigten Elektrodenoberfläche zu bilden,
wobei der mehrschichtige Film eine lose äußere Schicht aus Polyalkohol und eine innere stabile Schicht aus Polyalkohol aufweist,
(d) Abtragen der losen äußeren Schicht aus Polyalkohol und
(e) Durchführen der Oligonukleotidsynthese auf der inneren stabilen Schicht aus Polyalkohol.

4. Verfahren nach Anspruch 3, wobei die Elektrodenoberfläche Platin, Titan oder eine Kombination davon aufweist.

5. Verfahren nach Anspruch 3 oder 4, wobei die Elektrodenoberfläche eine Oberfläche eines Halbleiterchips ist.

6. Verfahren nach Anspruch 5, wobei der Halbleiterchip ein komplementärer Metalloxid-Halbleiter (CMOS) Chip ist.

7. Verfahren nach einem der Ansprüche 3 bis 6, wobei die Reinigung in (a) unter Verwendung eines Plasmas und/oder einer Verbindung durchgeführt wird.

8. Verfahren nach Anspruch 7, wobei das Plasma ausschließlich ein Sauerstoffplasma, ausschließlich ein Argonplasma, ein Sauerstoffplasma gefolgt von Argonplasma oder ein Argonplasma gefolgt von Sauerstoffplasma ist.

9. Verfahren nach einem der Ansprüche 7-8, wobei die Verbindung eine Piranha-Lösung, eine Säure, eine Base, Wasserstoffperoxid oder eine Kombination davon ist.

10. Verfahren nach Anspruch 3, das ferner die elektrochemische Reinigung der plasmagereinigten Elektrodenoberfläche vor Schritt (b) aufweist.

11. Verfahren nach Anspruch 10, wobei die elektrochemische Reinigung in Gegenwart von p-Toluolsulfonsäure durchgeführt wird.

12. Verfahren nach einem der Ansprüche 3 bis 11, wobei das Diazoniumsalz die Formel
Y-R-Ar-N₂⁺X⁻,
wobei:
Ar aus Phenyl, Xylyl, Naphthyl, Tolyl und Indolyl ausgewählt ist;
Y -OH, -COOH oder -NH2 ist, optional geschützt;
R aus einer Alkylgruppe, einer Alkenylgruppe, einer Arylgruppe und einer Heteroarylgruppe ausgewählt ist; und
X ein Halogen, Tetrafluorborat oder Tosylat ist.

13. Verfahren nach Anspruch 12, wobei das Diazoniumsalz aus der Gruppe ausgewählt ist, bestehend aus 4-(Hydroxymethyl)benzoldiazonium-tetrafluorboratsalz (AB-OH-Diazoniumsalz), 4-(2-Hydroxyethyl)benzoldiazoniumtetrafluorboratsalz (APE-OH-Diazoniumsalz) und 4-Carboxybenzoldiazoniumtetrafluorboratsalz (AB-Säure-Diazoniumsalz).

14. Verfahren nach einem der Ansprüche 3 bis 13, wobei der Polyalkohol Polybenzylalkohol, Polyazobenzolalkohol oder eine Kombination davon ist.

15. Verfahren nach einem der Ansprüche 3 bis 14, wobei das Abtragen der losen äußeren Schicht des Polyalkohols in Gegenwart von Ethylendiamin (EDA) und Ethanol, Ethanol, Aceton, Dimethylformamid (DMF), Ammoniumhydroxid, Dimethylsulfoxid (DMSO), Methanol oder einer Kombination davon durchgeführt wird.

## Revendications

1. Système de support solide pour la synthèse d'oligonucléotides, dans lequel le support est une électrode revêtue d'un film mince comprenant du polyalcool,
dans lequel l'électrode est préparée par un procédé comprenant
(a) le nettoyage de la surface de l'électrode,
(b) l'introduction d'un sel de diazonium sur la surface d'électrode nettoyée,
(c) l'électrogreffage du sel de diazonium de manière à former un film multicouche de polyalcool sur la surface d'électrode nettoyée,
dans lequel le film multicouche comprend une couche externe lâche du polyalcool et une couche interne stable du polyalcool,
(d) l'élimination de la couche externe lâche du polyalcool, et
(e) l'obtention de la couche interne stable du polyalcool pour la synthèse d'oligonucléotides.

2. Système de support solide selon la revendication 1, dans lequel le film mince est formé par électrogreffage de sels d'aryldiazonium fonctionnalisés ayant la formule :
Y-R-Ar-N₂⁺X⁻ ,
dans laquelle :
Ar est sélectionné parmi le phényle, le xylyle, le naphtyle, le tolyle et l'indolyle ;
Y est -OH, -COOH, ou -NH₂ éventuellement protégé ;
R est sélectionné parmi un groupe alkyle, un groupe alcényle, un groupe aryle et un groupe hétéroaryle ; et
X est un halogène, un tétrafluoroborate ou un tosylate.

3. Procédé de préparation d'une surface d'électrode pour la synthèse d'oligonucléotides, comprenant
(a) le nettoyage de la surface de l'électrode,
(b) l'introduction d'un sel de diazonium sur la surface d'électrode nettoyée,
(c) l'électrogreffage du sel de diazonium de manière à former un film multicouche de polyalcool sur la surface d'électrode nettoyée,
dans lequel le film multicouche comprend une couche externe lâche de polyalcool et une couche interne stable de polyalcool,
(d) l'élimination de la couche externe lâche de polyalcool, et
(e) la réalisation de la synthèse d'oligonucléotides sur la couche interne stable de polyalcool.

4. Procédé selon la revendication 3, dans lequel la surface d'électrode comprend du platine, du titane, ou une combinaison de ceux-ci.

5. Procédé selon la revendication 3 ou 4, dans lequel la surface d'électrode est une surface d'une puce à semi-conducteurs.

6. Procédé selon la revendication 5, dans lequel la puce à semi-conducteurs est une puce à oxyde de métal complémentaire (CMOS).

7. Procédé selon l'une quelconque des revendications 3 à 6, dans lequel le nettoyage en (a) est effectué en utilisant un plasma et/ou un composé.

8. Procédé selon la revendication 7, dans lequel le plasma est un plasma d'oxygène uniquement, un plasma d'argon uniquement, un plasma d'oxygène suivi d'un plasma d'argon, ou un plasma d'argon suivi d'un plasma d'oxygène.

9. Procédé selon l'une quelconque des revendications 7 à 8, dans lequel le composé est une solution piranha, un acide, une base, du peroxyde d'hydrogène, ou une combinaison de ceux-ci.

10. Procédé selon la revendication 3, comprenant en outre le nettoyage électrochimique de la surface d'électrode nettoyée par plasma avant l'étape (b).

11. Procédé selon la revendication 10, dans lequel le nettoyage électrochimique est effectué en présence d'acide p-toluènesulfonique.

12. Procédé selon l'une quelconque des revendications 3 à 11, dans lequel le sel de diazonium comprend la formule :
Y-R-Ar-N₂⁺X⁻,
dans laquelle :
Ar est sélectionné parmi le phényle, le xylyle, le naphtyle, le tolyle et l'indolyle ;
Y est -OH, -COOH, ou -NH₂, éventuellement protégé ;
R est sélectionné parmi un groupe alkyle, un groupe alcényle, un groupe aryle et un groupe hétéroaryle ; et
X est un halogène, un tétrafluoroborate ou un tosylate.

13. Procédé selon la revendication 12, dans lequel le sel de diazonium est sélectionné dans le groupe constitué du sel de tétrafluoroborate de 4-(hydroxyméthyl)benzènediazonium (sel de diazonium AB-OH), du sel de tétrafluoroborate de 4-(2-hydroxyéthyl)benzènediazonium (sel de diazonium APE-OH), et du sel de tétrafluoroborate de 4-carboxybenzènediazonium (sel de diazonium AB-acide).

14. Procédé selon l'une quelconque des revendications 3 à 13, dans lequel le polyalcool est du polyalcool benzylique, du polyalcool azobenzénique, ou une combinaison de ceux-ci.

15. Procédé selon l'une quelconque des revendications 3 à 14, dans lequel l'élimination de la couche externe lâche de polyalcool est effectuée en présence d'éthylènediamine (EDA) et d'éthanol, d'éthanol, d'acétone, de diméthylformamide (DMF), d'hydroxyde d'ammonium, de diméthylsulfoxyde (DMSO), de méthanol, ou d'une combinaison de ceux-ci.
